# NEW EUROPEAN PATENT SPECIFICATION

(11) **EP 0 649 541 B2**
(45) Date of publication and mention of the opposition decision: **23.01.2002**
(45) Mention of the grant of the patent: 10.09.1997
(21) Application number: 93914635.3
(22) Date of filing: 28.06.1993
(51) Int. Cl.: G02C 13/00, A61L 2/18, A61L 12/08, A61L 12/12

(54) **METHOD AND CONTAINER FOR DISINFECTION OF CONTACT LENSES**
VERFAHREN UND BEHÄLTER FÜR DIE DESINFEKTION VON KONTAKTLINSEN
PROCEDE ET RECIPIENT DE DESINFECTION DE VERRES DE CONTACT

(30) Priority: 06.07.1992 DK 88592
(43) Date of publication of application: 26.04.1995
(73) Proprietor: NIELSEN, Tom Buris, DK-8250 Egaa (DK)
(72) Inventor: NIELSEN, Tom Buris, DK-8250 Egaa (DK)
(74) Representative: Gregersen, Niels Henrik
(86) International application number: PCT/DK93/00211
(87) International publication number: WO 94/01800

(56) References cited:
- EP-A- 0 209 071
- ES-A- 2 022 020
- US-A- 4 011 941
- US-A- 4 826 658
- US-A- 5 011 661

## Description

The present invention relates to a method for disinfection of contact lenses of the type described in the introductory part of claim 1.

Users of contact lenses, particularly soft contact lenses, are familar with the fact that contact lenses frequently, preferably daily, have to be cleaned to avoid inconveniences by the use of contact lenses.

Normally hydrogen peroxide is used for disinfection of the contact lenses, which for this purpose is placed in special cases, which may be used for the keeping of the contact lenses as well as for disinfection of these. The contact lenses are kept in the cleaning fluid (hydrogen peroxide) until next use, for instance through the night.

In order to neutralize the hydrogen peroxide before next use of the contact lenses, it is known to neutralize the hydrogen peroxide - by using of a "platinum star", which acts as a catalyst, or by using an enzyme tablet - containing for instance catalase - to neutralize the hydrogen peroxide.

US-A-4 011 941 discloses an apparatus comprising a cleaning capsule for the sterilizing of soft contact lenses in an aqueous hydrogen peroxide solution and a method where the neutralizing agent is brought into contact with the cleaning solution by inverting the cleaning capsule - and where use is made of a catalytic reactor coated with a layer of platimum.

To ensure that there will be time enough for the hydrogen peroxide to disinfect the contact lenses for micro organism and virus before the neutralization, it is furthermore known to delay the effect of the catalase by using a special tablet, where the catalase is coated with methyl-cellulose or a PVP-film. EP-A-0 209 071 discloses the use of such an encapsulated neutralizing tablet which is inserted into the cleaning solution and releases the neutralizing agent with delay. ES-A-2 022 020 discloses a method for disinfection of contact lenses making use of a closed, perforated comportment for the neutralizing agent. The comportment constitutes a physical impediment for the entramer of the cleaning fluid.

Catalase is an enzyme, which is capable to convert the hydrogen peroxide into water and free oxygen. In practise the used tablet contains a salt with catalase, so that the hydrogen peroxide is converted into physilogical salt water.

The purpose of the invention is to provide a method enabling a further improvement by disinfection of the contact lenses cf. the method as mentioned introductorily.

The method according to the invention is defined in claim 1 and is distinctive in that disinfection of the contact lenses and neutralization of the cleaning fluid, respectively, are controlled by means of a tablet containing the enzyme catalase, said tablet is placed in a separate chamber, and that the admission for the cleaning fluid to this chamber is controlled by the generation of oxygen by the reaction between the cleaning fluid and the enzyme catalase.

Preferably the method according to the invention is such modified that use is made of a tablet containing the enzyme catalase without being encapsulated or coated.

In a simple manner it hereby becomes possible to control the duration of the disinfection as well as the neutralization of the cleaning fluid by using a simple catalase tablet, which does not need any coating, that is that a rather cheap catalase tablet may be used for control of a slowly, delayed neutralization of the cleaning fluid.

The invention furthermore relates to a container or case for use in the method according to the invention, which contain er is distinctive in that it comprises a chamber with a room for a right contact lens and a room for a left contact lens, and a chamber which is adapted to receive a catalase tablet, said chamber for the contact lenses being connected to said chamber for the catalase tablet in such a manner that the admission of the cleaning fluid to the neutralization agent is controlled.

The case is such provided, that an opening is provided between said chamber for the contact lenses and said chamber for the catalase tablet, said opening for free access of fluid being placed in a level just under the tablet in the chamber, the position of said opening being adapted to control the speed of the neutralization by controlling the oxygen stream from said chamber, respectively to the chamber for the contact lenses.

The invention is described in the following with reference to the drawing, in which:
- Fig. 1: shows a sectional view through a preferred embodiment for a case according to the invention - shown with contact between tablet and cleaning fluid,
- Fig. 2: shows a similar sectional view of. Fig. 1, but shown in a situation, where the cleaning fluid because of the air generation is forced away from the tablet,
- Fig. 3: shows the case - seen from above and without a lid part,
- Fig. 4: shows a side sectional view of a lid part with a holding cavity for the neutralization tablet,
- Fig. 5: shows a sectional view through another embodiment for a case according to the invention, shown in a situation, where an air bubble is keeping the cleaning fluid away from the tablet,
- Fig. 6: shows a similar sectional view cf. Fig. 5, but shown in a situation, where the air bubble is escaping and permits access of fresh cleaning fluid to the tablet,
- Fig. 7: shows the case cf. Figs. 5 and 6 - seen from above and without a lid part,
- Fig. 8: shows an end view of a known case for keeping and cleaning contact lenses and provided with a combined lens and tablet holder using the method according to the invention, and
- Fig. 9: shows a side view of the combined lens and tablet holder cf. Fig. 8.

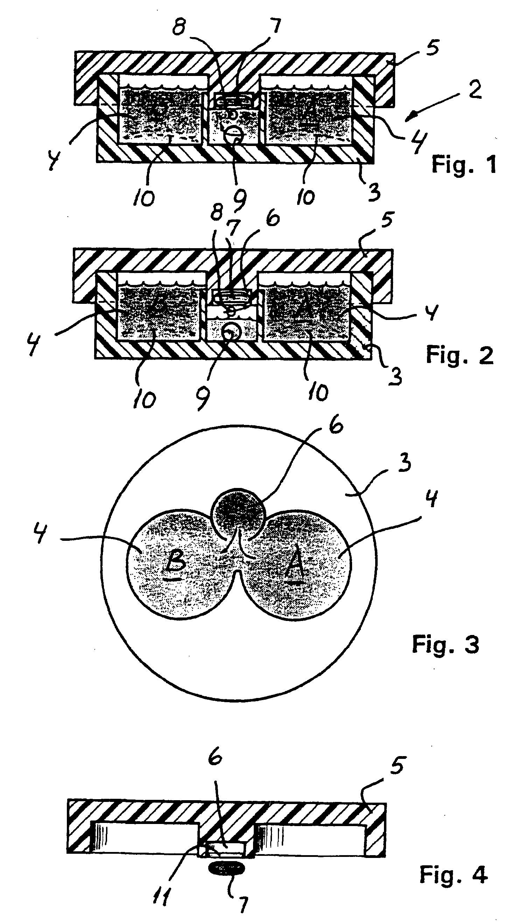

A case 2, shown in Figs. 1 - 3, consists of a lower part 3 with a chamber with a room A for a right contact lens and a room B for a left contact lens and a lid part 5 (Fig. 4) with a chamber 6 adapted to keep or fix a tablet 7 containing catalase or another enzyme.

A pair of contact lenses 10 to be cleaned or disinfected is placed in the double chamber 4, and cleaning fluid in the form of hydrogen peroxide (Fig. 1) is filled in. A catalase tablet 7 is squeezed in the chamber 6 of the lid part. The chamber 6 has for that purpose a projecting, lower edge 11, behind which the tablet 7 is squeezed, before the lid part 5 is again placed on the lower part 3.

By contact between the catalase tablet 7 and the hydrogen peroxide free oxygen is generated, and an air bubble (Fig. 2) is formed around respectively under the tablet 7, so that the generation of oxygen is reduced. In the side of the chamber 6 an opening 8 is provided into the double chamber 4, through which opening 8 the oxygen bubble may escape. Afterwards the hydrogen peroxide gets re-admission to the catalase tablet 7, and a new portion of oxygen is generated and escaping through the opening 8 or escapes otherwise - and this procedure continues until the tablet 7 is dissolved.

Eventually the lower part 3 on level with the chamber 6 in the lid part 5 may comprise a complementary part, which is provided with a side opening 9 in order to improve the circulation between the rooms of the double chamber-around the contact lenses 10 (Figs. 1 and 2).

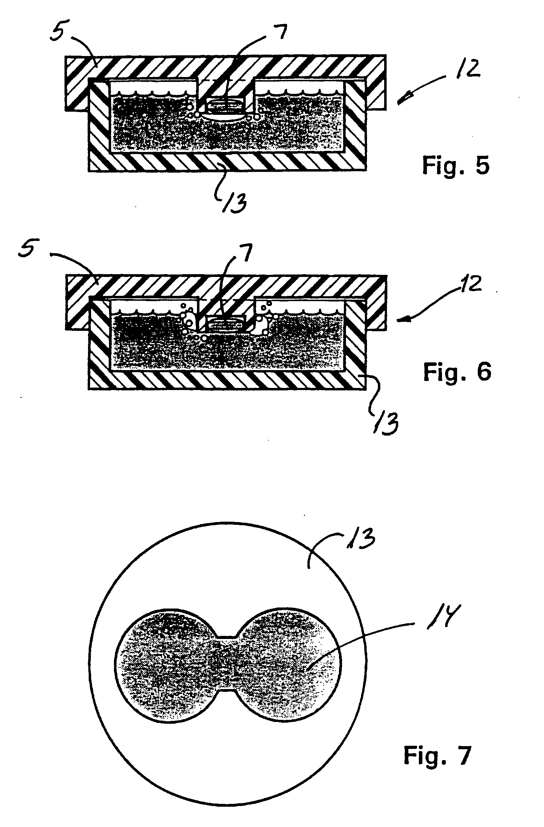

An other embodiment of a case 12 according to the invention - shown in Figs. 5 - 6 - comprises a lower part 13 having a double chamber 14 with two positions for a pair of soft contact lenses and a lid part 5 with a similar configuration as that of the lid part 5 shown in Fig. 4.

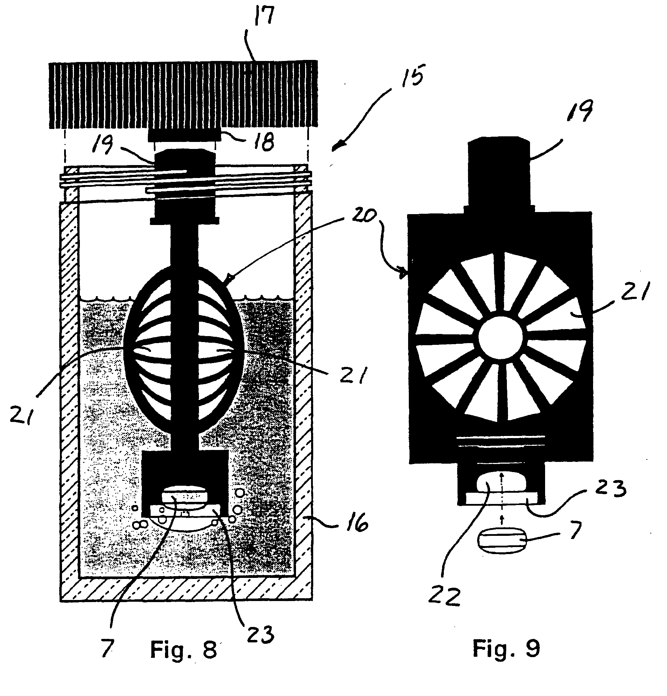

In Fig. 8 Is shown a known case 15 comprising a lower part 16 and a screw lid 17 with a central socket 18 for a top end portion 19 of a holding member 20 with two basket-like chambers 21 adapted to keep a pair of contact lenses - as the holding member 20 is submerged into cleaning fluid. At the bottom end of the holding member 20 a special cavity 22 or position with a collar 23 adapted to receive and fix a catalase tablet 7 is provided. The holding member 20 is shown from the side in Fig. 9,

## Claims

1. A method for disinfection of contact lenses comprising the steps of placing the contact lenses (10) in a cleaning fluid in the form of hydrogen peroxide in a container, and carrying out neutralization of the cleaning fluid by means of a tablet (7) containing a neutralization agent catalase, ***characterized* by** the step of placing said tablet containing the enzyme catalase, in a separate chamber (6) within said container, said chamber having an opening (8) for free access of fluid being placed in a level just under the tablet, such that the admission for the cleaning fluid to this chamber is controlled by the generation of oxygen by the reaction between the cleaning fluid and the enzyme catalase, whereby disinfection of the contact lenses (10) and neutralization of the cleaning fluid, respectively, are controlled by means of said tablet (7).

2. A method according to claim 1, ***characterized* in that** use is made of a tablet containing the enzyme catalase without being encapsulated or coated.

3. A container for use in the method according to claim 1, said container comprising a chamber (4) with a room (A) for a right contact lens (10) and a room (B) for a left contact lens, and a chamber (6) which is adapted to receive said enzyme catalase tablet, said chamber having an opening (8) for free access of fluid being placed in a level just under the tablet, said chamber (4) for the contact lenses (10) being connected to said chamber (6) for the catalase tablet (7) in such a manner that the admission of the cleaning fluid to the neutralization agent is controlled by the generation of oxygen by the reaction between the cleaning fluid and the enzyme catalase tablet.

## Patentansprüche

1. Verfahren zur Desinfektion von Kontaktlinsen, bei dem die Kontaktlinsen (10) in eine Reinigungsflüssigkeit in Form von Hydrogenperoxid in einen Behälter eingesetzt werden und die Neutralisation der Reinigungsflüssigkeit durch eine Tablette (7) mit einem neutralisierenden Katalaseagens durchgerührt wird, **dadurch gekennzeichnet dass** die Tablette mit der Enzymkatalase in eine separate Kammer (6) in dem Behälter eingesetzt wird, wobei die Kammer eine Öffnung (8) für den freien Zutritt von Flüssigkeit in einer Ebene gerade unter der Tablette aufweist, so dass der Zutritt der Reinigungsflüssigkeit zu dieser Kammer durch die Erzeugung von Sauerstoff aufgrund Reaktion zwischen der Reinigungsflüssigkeit und der Enzymkatalase gesteuert wird, wodurch die Desinfektion der Kontaktlinsen (10) bzw. die Neutralisation der Reinigungsflüssigkeit durch die Tablette (7) gesteuert werden.

2. Verfahren nach Anspruch 1, **dadurch gekennzeichnet, dass** eine Tablette verwendet wird, die die Enzymkatalase kapselfrei oder ohne Beschichtung aufweist.

3. Behälter zur Verwendung bei einem Verfahren nach Anspruch 1, bei dem der Behälter eine Kammer (4) mit einem Raum (A) für eine rechte Kontaktlinse (10) und einen Raum (B) für eine linke Kontaktlinse und eine Kammer (6) aufweist, die so ausgebildet ist, dass sie eine Enzymkatalasetablette aufnehmen kann, wobei die Kammer eine Öffnung (8) für den freien Zutritt von flüssigkeit in einer Ebene gerade unter der Tablette aufweist, wobei die Kammer (4) für die Kontaktlinsen (10) mit der Kammer (6) der Katalasetablette (7) so verbunden ist, dass der Zutritt der Reinigungsflüssigkeit zum Neutralisationsagens durch die Erzeugung von Sauerstoff durch Reaktion zwischen der Reinigungsflüssigkeit und der Enzymkatalasetablette gesteuert wird.

## Revendications

1. Procédé de désinfection de lentilles de contact, comprenant les étapes consistant à placer les lentilles de contact (10) dans un liquide nettoyant sous la forme de peroxyde d'hydrogène dans un conteneur et à effectuer la neutralisation du liquide nettoyant au moyen d'un comprimé (7) contenant une catalase comme agent de neutralisation, **caractérisé par** l'étape consistant à placer ledit comprimé contenant l'enzyme catalase dans une chambre séparée (6) à l'intérieur dudit conteneur, ladite chambre comprenant une ouverture (8) permettant le libre accès du liquide étant situé à un niveau situé juste au-dessous du comprimé de telle façon que l'admission du liquide nettoyant à cette chambre soit maîtrisée par la formation d'oxygène au moyen d'une réaction entre le liquide nettoyant et l'enzyme catalase, moyennant quoi on maîtrise respectivement la désinfection des lentilles de contact (10) et la neutralisation du liquide nettoyant au moyen dudit comprimé (7).

2. Procédé selon la revendication 1, **caractérisé en ce qu'**on utilise un comprimé contenant l'enzyme catalase sans encapsulation et sans enrobage.

3. Conteneur destiné à être utilisé dans le procédé selon la revendication 1, ledit conteneur comprenant une chambre (4) avec un compartiment (A) pour une lentille de contact droite (10) et un compartiment (B) pour une lentille de contact gauche, et une chambre (6) qui est conçue pour recevoir ledit comprimé d'enzyme catalase, ladite chambre comprenant une ouverture (8) permettant le libre accès du liquide étant situé à un niveau situé juste au-dessous du comprimé, ladite chambre (4) pour les lentilles de contact (10) étant reliée à ladite chambre (6) pour le comprimé (7) d'enzyme catalase de telle façon que l'admission du liquide nettoyant à l'agent de neutralisation soit maîtrisée par la formation d'oxygène au moyen d'une réaction entre le liquide nettoyant et le comprimé d'enzyme catalase.
